# EUROPÄISCHE PATENTANMELDUNG

(11) **EP 0 645 157 A2**
(43) Veröffentlichungstag der Anmeldung: **29.03.1995**
(21) Anmeldenummer: 94250227.9
(22) Anmeldetag: 13.09.1994
(51) Int. Cl.: A61M 11/06, A61M 16/16

(54) **Flasche zur Anfeuchtung von Atemgas**

(30) Priorität: 24.09.1993 DE 4332581
(71) Anmelder: KENDALL MEDIZINISCHE ERZEUGNISSE GmbH, D-93333 Neustadt (DE)
(72) Erfinder: Welnhofer, Werner, D-93326 Abensberg (DE); Kopp, Rudolf, D-93309 Kelheim (DE)
(74) Vertreter: UEXKÜLL & STOLBERG

(57) **Zusammenfassung**

Die Erfindung betrifft eine Flasche (1) für das Anfeuchten von Atemgas. Eine Steigleitung (6) führt an der Rückwand (4) der Flasche von deren oberem Ende zu deren Boden (5) und erstreckt sich mit einem horizontalen Teil (6') entlang dem Boden (8). Der horizontale Teil (6') der Steigleitung (6) weist kleine Löcher (10) auf, die sich durch den Boden (8) der Flasche (1) erstrecken und damit eine Strömungsverbindung vom Inneren der Flasche (1) zur Steigleitung (6) herstellen.

## Beschreibung

Die Erfindung betrifft eine Flasche gemäß dem Oberbegriff des Patentanspruchs 1.

Aus der **US-PS 3,903,216** is bereits eine derartige Flasche bekannt, die an einer Seite einen vertikalen Kanal aufweist, der entlang der Flasche von oben nach unten verläuft. Der vertikale Kanal ist vor dem Gebrauch an seinem oberen Ende geschlossen, an dem er ein Außengewinde für die Herstellung einer Schraubverbindung aufweist. Das untere Ende mündet in den Innenraum der Flasche. Der Hauptkörper der Flasche weist einen rechtwinkelig eingesetzten Stutzen auf, der ebenfalls vor dem Gebrauch durch eine dünne Membran verschlossen ist. Auch dieser Stutzen ist mit einem Außengewinde für die Herstellung eines Schlauchanschlusses versehen. Die bekannte Flasche wird vor dem Gebrauch mit sterilen Wasser gefüllt. Im Gebrauch werden an das vertikale Steigrohr und an den abgewinkelten Stutzen Leitungen angeschlossen und es wird Atemgas durch das Wasser gedrückt, daß auf dieser Weise befeuchtet wird. Ein Nachteil der bekannten Flasche besteht darin, daß das Wasser am unteren Ende des senkrecht Steigrohres durch einen verhältnismäßig großen Querschnitt aus- oder eintritt, so daß es in großen Blasen durch die Flasche perlt.

**Aufgabe** der Erfindung ist es, die bekannte Flasche dahingehend weiterzubilden, daß eine bessere Beladung des Atemgases mit Wasser erreicht wird.

Zur **Lösung** dieser Aufgabe dienen die Merkmale des Patentanspruchs 1.

Die Erfindung wird im folgenden anhand von Figuren näher erläutert; es zeigen:
**Figur 1** eine Seitenansicht der Ausführungsform;
**Figur 2** eine Ansicht der Ausführungsform; und
**Figur 3** eine Daraufsicht auf die Ausführungsform nach den Figuren 1 und 2.

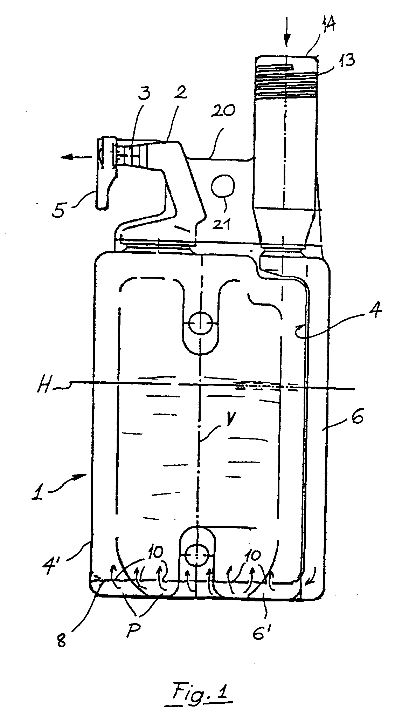

**Figur 1** zeigt eine Flasche 1, zweckmäßigerweise aus Kunststoff, die an ihrem oberen Ende einen abgewinkelten Anschlußstutzen 2 aufweist, der etwa die Form eines "Hundebeins" hat. Er dient dazu, eine Schlauchleitung parallel zur horizontalen Achse H der Flasche 1 anzuschließen. Zu diesem Zweck ist der Anschlußstutzen 2 mit einer Riffelung 3 versehen, die den nicht dargestellten Schlauch halten soll. Es ist klar, daß der Schlauch mit einer Klemmverbindung, beispielsweise mit einer Schelle am Anschlußstutzen 2 gehalten wird. Vor dem Gebrauch ist das vordere Ende des Anschlußstutzens 2 durch eine angeformte Kappe 5 verschlossen.

An einer Seite der Flasche 1, die hier als Rückwand 4 bezeichnet sei, ist außen eine Steigleitung 6 angeformt, die entlang der Rückwand 4 keine Strömungsverbindung zum Innenraum der Flasche 1 hat. Die Steigleitung 6 führt jedoch zum Teil um die Flasche 1 herum, indem sie sich L-förmig unter den Boden 8 der Flasche erstreckt und mit diesem ebenso wie mit der Rückwand 4 verbunden ist. Der horizontale Teil 6' der Steigleitung 6 endet an der Vorderwand 4' der Flasche 1. Wesentlich ist es nun für die Erfindung, daß der horizontale Teil 6' der Steigleitung 6 kleine Löcher 10 oder Perforationen aufweist, die sich durch den Boden 8 und durch die Wand der Steigleitung 6' erstrecken, so daß eine Strömungsverbindung von der Steigleitung 6 zum Innenraum der Flasche 1 hergestellt ist. Dies ist durch die Pfeile P angedeutet.

Die Steigleitung 6 weist an ihrem oberen Ende in der Nähe des Anschlußstutzens 2 ein Anschlußstück 12 auf, daß sich parallel zur vertikalen Achse V der Flasche 1 erstreckt und an seinem oberen Ende mit einem Gewinde 13 für das Aufschrauben einer Schlauchverbindung versehen ist. Vor dem Gebrauch ist das Anschlußstück 12 im übrigen an seiner Oberseite durch eine Membran 14 verschlossen. Steriles Wasser ist dabei natürlich zuvor in der Flasche 1 eingefüllt worden.

Im Gebrauch werden zunächst die Anschlußverbindungen hergestellt, indem Luftleitungen an den Anschlußstutzen 2 und an das Anschlußstück 12 angeschlossen werden. Dabei ist zuvor die Kappe 5 von Anschlußstutzen 2 abzutrennen beziehungsweise die Membran 14 am Anschlußstück 12 zu durchstechen. Nun kann Luft durch das Anschlußstück 12 eingeleitet werden und strömt dann zunächst noch getrennt vom Innenraum der Flasche 1 durch den vertikalen Teil der Steigleitung 6 nach unten bis unter dem Boden 8 der Flasche 1, um durch die kleinen Löchern 10 aus dem horizontalen Teil 6' der Steigleitung 6 in die Flasche 1 einzudringen und dort durch das sterile Wasser nach oben in Richtung auf den Anschlußstutzen 2 zu perlen. Durch die Anordnung der kleinen Löcher 10 läßt sich das Gas in kleine Perlen aufbrechen und reißt damit verhältnismäßig viel Wasser mit.

Es wird darauf hingewiesen, daß der Gasstrom auch umgekehrt erfolgen kann, indem das Einströmen durch den Anschlußstutzen 2 und das Abströmen durch das Anschlußstück 12 erfolgt.

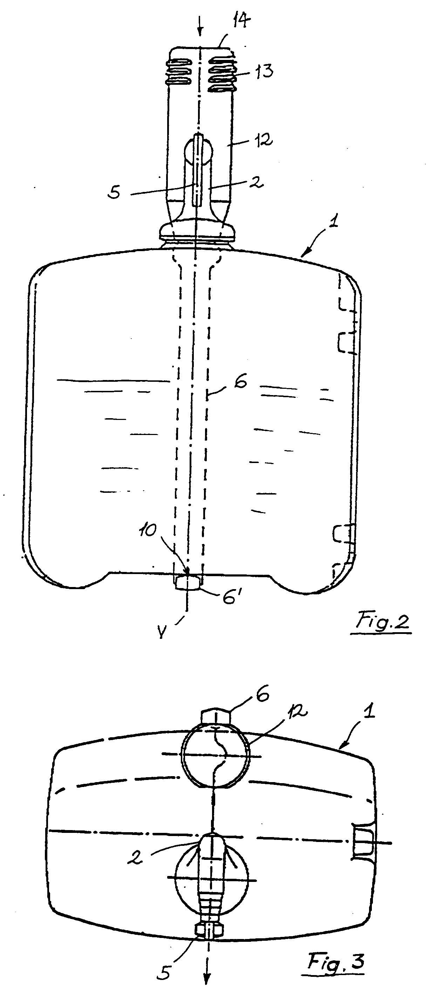

Die **Figuren 2 und 3** erläutern diese Strömungsverhältnisse, wobei gleiche Teile mit gleichen Bezugszeichen versehen sind.

Der Anschlußstutzen 2 und das Anschlußstück 12 sind außerdem durch einen Steg 20 verbunden, der ihnen Stabilität verleiht und durch eine Öffnung 21 außerdem die Möglichkeit bietet, die Flasche 1 aufzuhängen.

## Patentansprüche

1. Flasche zur Anfeuchtung von Atemgas mit sterilem Wasser, mit einem an der Oberseite vorgesehenen, abgewinkelten Anschlußstutzen (2), der vor dem Gebrauch verschlossen ist, und mit einer an der Rückwand (4) außen angebrachten Steigleitung (6), die an ihrem oberen Ende ein Anschlußstück (12) aufweist und an ihrem unteren Ende mit dem Inneren der Flasche (1) in Strömungsverbindung steht, **dadurch gekennzeichnet**, daß die Steigleitung (6) einen horizontalen Teil (6') aufweist, der sich unter dem Boden (8) der Flasche (1) erstreckt, mit diesem verbunden ist und über eine Anzahl von kleinen Löchern (10) die Strömungsverbindung von Innenraum der Flasche 1 zum Inneren der Steigleitung (6) herstellt.

2. Flasche nach Anspruch 1, **dadurch gekennzeichnet**, daß sie aus Kunststoff hergestellt ist.

3. Flasche nach einem der Ansprüche 1 oder 2, **dadurch gekennzeichnet**, daß die Steigleitung (6, 6') an die Flasche angeformt ist.

4. Flasche nach einem der Ansprüche 1 bis 3, **dadurch gekennzeichnet**, daß der Anschlußstutzen (2) und das Anschlußstück (12) durch einen Steg (20) verbunden sind.

5. Flasche nach Anspruch 4, **dadurch gekennzeichnet**, daß der Steg (20) eine Öffnung (21) aufweist.
